Europäisches Patentamt

European Patent Office

Office européen des brevets

(11) Veröffentlichungsnummer: **0 289 938**
**A1**

## EUROPÄISCHE PATENTANMELDUNG

(21) Anmeldenummer: 88106800.1

(22) Anmeldetag: 28.04.88

(51) Int. Cl.⁴ **A63B 21/00 , A63B 23/04**

(30) Priorität: 06.05.87 DE 3714996

(43) Veröffentlichungstag der Anmeldung:
**09.11.88 Patentblatt 88/45**

(84) Benannte Vertragsstaaten:
**AT BE CH ES FR GB GR IT LI LU NL SE**

(71) Anmelder: **Hamm, Wilfried**
**Im Wiesengrund 28**
**D-4330 Mülheim-Ruhr(DE)**

(72) Erfinder: **Hamm, Wilfried**
**Im Wiesengrund 28**
**D-4330 Mülheim-Ruhr(DE)**

(54) **Steuerung für Heimtrainer.**

㊗ Gegenstand der Erfindung ist eine Steuerung für Heimtrainer, die aus einem programmierbaren Microcomputer besteht. Der Microcomputer wird mit Programmen und Daten per Magnetkarten oder von anderen Massenspeichern gespeist und ermöglicht somit genau vorgegebene Belastungsintervalle. Eingesetzt in der Rehabilitationstherapie kann der Heimtrainer mit der genannten Steuerung zu einem Behandlungsgerät werden, bei dem keine Dauerkontrolle des Artzes notwendig ist. Ermöglicht wird dieses durch die Eingabe der Programme und Daten mittels Magnetkarten o.ä., die keinen nachträglichen Eingriff durch den Patienten zulassen.

EP 0 289 938 A1

## Steuerung für Heimtrainer

Die Erfindung betrifft eine Steuerung für Heimtrainer. Derartige Steuerungen arbeiten zumeist mit einem Microcomputer, der ein fest eingegebenes Programm besitzt, welches zur Ergänzung nur noch mit den persönlichen Daten sowie der erwünschten Pulsobergrenze des Trainierenden gefüttert werden muß. Dieses geschieht direkt am angebauten Microcomputer durch den Trainierenden selbst. Entsprechend der eingegebenen Daten wird dann das Programm abgearbeitet. Dabei wird dann ein Regelkreis aktiviert, der aus der gemessenen Pulsfrequenz entsprechend auf die Wirbelstrombremse des Heimtrainers wirkt.

In der Hand des Arztes wird ein solcher Heimtrainer zum Instrument für die Rehabilitationstherapie bzw. Ergo-Therapie. Die Anwendung bei kreislaufgeschädigten Patienten muß dann unter ärztlicher Aufsicht stattfinden, damit die Patienten durch evtl. Übereifer nicht zu höheren Vorgabewerten, als der Arzt sie vorgibt, übergehen und sich damit gesundheitlichen Schaden zufügen. Diese bisherige Anwendung unter dauernder Aufsicht des Arztes ist zweifellos aufwendig und teuer sowie für die Patienten umständlich.

Aufgabe der Erfindung ist es, eine sicher durchzuführende Therapie ohne direkte Aufsicht des Arztes zu ermöglichen.

Die Aufgabe wird dadurch gelöst, daß ein Microcomputer mit Daten und Trainingsprogrammen von Magnetkarten oder sonstigen Massenspeichern programmiert wird, die eine genaue Vorgabe der Belastung und ihrer Intervalle ermöglicht. Dabei können die Programme und Daten nur durch das dazu befugte Personal geändert werden.

Eine solche Vorgehensweise ermöglicht es, den Heimtrainer leihweise in die Wohnung des Patienten zu geben, wobei der Microcomputer vom Arzt für diesen speziellen Anwendungsfall programmiert wurde und der Patient keine Möglichkeit besitzt, daran etwas zu manipulieren. Der Patient braucht dann lediglich von Zeit zu Zeit beim Arzt vorzusprechen und ihm die Kontrollaufzeichnungen des kleinen angebauten Druckers zu zeigen. Je nach Fortschritt des Patienten kann diesem dann beispielsweise eine Magnetkarte mit geänderten Belastungsintervallprogrammen mitgegeben werden, die er dann selbst durch den Kartenleser des Microcomputers durchschiebt. Selbst vom Arzt zeitlich vorgegebene Belastungsintervalle sind möglich. Die beiden einprogrammierten Parameter, Datum und Uhrzeit, geben den Programmablauf erst bei eintreffen dieser frei.

Aber auch für die reine Verwendung des Trainers in Therapiezentren ergeben sich vielfältige Möglichkeiten einer Belastungs-Intervallprogrammierung, wobei die verschiedenen Programme auf Massenspeichern oder Magnetkarten abgelegt sind und vom Bedienungspersonal leicht eingegeben werden können.

Dadurch wird die Therapie vielfältiger und kostengünstiger und für den Reha-Patienten sicherer. Eine physische Überbelastung ist vollkommen ausgeschlossen.

## Ansprüche

1) Steuerung für Heimtrainer, dadurch gekennzeichnet, daß ein Microcomputer mit Daten und Trainingsprogrammen von Magnetkarten oder sonstigen Massenspeichern programmiert wird, die eine genaue Vorgabe der Belastung und ihre Intervalle ermöglicht.

2) Steuerung für Heimtrainer nach Anspruch 1, dadurch gekennzeichnet, daß die Programme und Daten der Microcomputer nur durch dazu befugtes Personal geändert werden kann.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 028 209 (SMIDAK)<br>* Seite 1, Zeilen 5-9; Seite 5, Zeilen 24-36; Seite 7, Zeilen 4-14; Seite 9, Zeilen 28-37; Seite 10, Zeilen 4-21; Seite 13, Zeilen 13-19 *<br>--- | 1,2 | A 63 B 21/00<br>A 63 B 23/04 |
| X | US-A-3 675 640 (GATTS)<br>* Spalte 1, Zeile 62 - Spalte 2, Zeile 22; Spalte 3, Zeilen 3-22,47-64; Spalte 6, Zeilen 31-36,70-73; Spalte 7, Zeilen 23-54 *<br>--- | 1,2 | |
| X | GB-A-1 350 068 (SIMPSON)<br>* Seite 1, Zeilen 33-78 *<br>--- | 1,2 | |
| X | DE-A-3 408 303 (SCHWEGLER)<br>* Seite 4, Zeilen 1-9; Seite 7, Zeile 4 - Seite 8, Zeile 13 *<br>--- | 1 | |
| A | BE-A- 897 474 (SPRINGAEL)<br>* Seite 1, Zeilen 1-19 *<br>----- | 2 | |

RECHERCHIERTE SACHGEBIETE (Int. Cl.4)

A 63 B

Der vorliegende Recherchenbericht wurde für alle Patentansprüche erstellt

| Recherchenort | Abschlußdatum der Recherche | Prüfer |
|---|---|---|
| DEN HAAG | 25-07-1988 | SCHOENLEBEN J.E.F. |

EPO FORM 1503 03.82 (P0403)